Europäisches Patentamt

European Patent Office

Office européen des brevets

(11) Publication number: **0 218 472**
**A2**

## (12) EUROPEAN PATENT APPLICATION

(21) Application number: **86307584.2**

(22) Date of filing: **02.10.86**

(51) Int. Cl.⁴: **C 12 N 9/02**

(30) Priority: **03.10.85 JP 219166/85**

(43) Date of publication of application:
**15.04.87 Bulletin 87/16**

(84) Designated Contracting States:
**AT BE CH DE ES FR GB GR IT LI LU NL SE**

(71) Applicant: **Suntory Limited**
**1-40 Dojimahama 2-chome Kita-ku**
**Osaka-shi Osaka-fu 530 (JP)**

(72) Inventor: **Asami, Sumio**
**16-16-9 Futaba-cho**
**Ibaraki-shi Osaka (JP)**

**Kusumi, Takaaki**
**15-C-402 Yamate-cho 3-chome**
**Suita-shi Osaka (JP)**

**Amachi, Teruo**
**1-10 Hibarigaokayamate 2-chome**
**Takarazuka-shi Hyogo (JP)**

**Yoshizumi, Hajime**
**6-1-612 Kosobe-cho 2-chome**
**Takatsuki-shi Osaka (JP)**

(74) Representative: **Ford, Michael Frederick**
**MEWBURN ELLIS & CO. 2/3 Cursitor Street**
**London EC4A 1BQ (GB)**

(54) **Process for production of superoxide dismutase.**

(57) A process for the preparation of superoxide dismutase by the fermentation method using yeast cells, which comprises subjecting yeast cells grown by culturing under an anaerobic condition to an aeration treatment or an aeration treatment with agitation to increase superoxide dismutase in the cells, and recovering the superoxide dismutase.

EP 0 218 472 A2

**Description**

## PROCESS FOR PRODUCTION OF SUPEROXIDE DISMUTASE

The present invention relates to a process for the preparation of superoxide dismutase (hereinafter referred to as "SOD"). More particularly, the present invention relates to a process for the preparation of SOD which comprises subjecting yeast cells grown under an anaerobic condition to an aeration treatment, or an aeration treatment with agitation, to increase the SOD content in yeast cells and recovering SOD from the yeast cells.

SOD is scavenging a superoxide anion radical ($O_2 \cdot$), represented by the following formula:
$2O_2 \cdot\ + 2H+ \rightarrow H_2O_2 + O_2$.
It is known that this enzyme is widely present in animals, plants and microorganisms (I. Fridovich, Adv. Enzymol., 41, 35-97, 1974).

It is proposed that the superoxide anion radical ($O_2 \cdot$) is an important agent of the toxicity of oxygen. As various physiological actions of the superoxide anion radical have been recently elucidated, the role of SOD as the biological defending enzyme has attracted attention.

For example, it has been found that SOD is effective for preventing oxidation of an easily oxidizable substance such as a food (Japanese Unexamined Patent Publication No. 50-40785), as a cosmetic component for protecting the skin and hair, preventing pigmentation on the skin, exerting an anti-inflammatory action on the skin or preventing deterioration of a component in a cosmetic composition (Japanese Unexamined Patent Publications No. 51-63949 and No. 55-87712), and for the clinical treatment of rheumatoid arthritis and the like (M. Walravens & J. Dequeker, Current Therapeutic Res., 20, 62-69, 1976).

It is generally considered that a microorganism will be used as a supply source of SOD when the mass production of SOD is attempted on an industrial scale, and a process has been proposed in which manganese-SOD is prepared from a microorganism belonging to the genus Serratia (Japanese Unexamined Patent Publication No. 57-29285). It is considered that when SOD is intended to be added to foodstuffs or cosmetics for attaining the above-mentioned effects, preferably a yeast of Saccharomyces cerevisiae, which has been long used for the preparation of fermentation foodstuffs and drinks and has been confirmed to have a high safety factor, is used as the supply source. A process for obtaining a large quantity of copper-zinc-SOD from baker's yeast is known (Japanese Unexamined Patent Publication No. 56-35983), but, in this prior art, there is no teaching of a way in which to increase the accumulation of SOD.

Regarding the relationship between the yeast culturing conditions and the SOD content in cells, it is reported that the content of SOD in cells is increased with an increase in the partial pressure of oxygen (E.M. Gregory et al, J. Bacteriol., 117, 456-460 1974), but this report does not disclose the growth of yeasts under anaerobic conditions. Large energy costs are necessary for aeration and agitation in the method in which yeast cells are obtained by aerobic culturing, and this method is not necessarily advantageous from the economical viewpoint.

In alcohol-forming fermentation such as the brewing of beer, large quantities of waste yeasts are discharged as by-products. These waste yeasts have been heretofore utilized for the production of dry yeast agents and seasoning liquids, but, desirably, they could be utilized as the starting material of products having a higher economical value.

Under the above technical background, it is a primary object of the present invention to provide a process for preparing SOD economically advantageously by utilizing relatively cheap yeasts grown under anaerobic conditions, such as waste yeasts discharged in alcohol-forming fermentation, for example, the brewing of beer.

Therefore, the present invention provides a new process for the preparation of superoxide dismutase by the fermentation method using yeast cells, which comprises subjecting yeast cells grown by culturing under anaerobic conditions to an aeration treatment, or an aeration treatment with agitation, to increase superoxide dismutase in the cells and recovering the superoxide dismutase.

In the present invention, yeast cells grown under anaerobic conditions are used. Any yeast strain can be used, so far as it is capable of accumulating SOD in a collectable quantity under aerobic conditions. In the present invention, however, it is preferable to use waste yeast cells discharged from the brewing process. From this viewpoint, a brewing strain, for example, beer yeast, wine yeast or sake yeast belonging to the genus Saccharomyces, is preferably used as the yeast strain.

Cells of the yeast used in the process of present invention can be prepared particularly for the purpose of the present invention. For example, the above-mentioned yeast is anaerobically cultured without aeration or agitation according to conventional procedures in an conventional culture medium for the culture of yeast, prepared by using sugar materials such as molasses. However, from the economical viewpoint, preferably waste yeast cells discharged in a large quantity as a by-product in the brewing process are used. As the waste yeast cells, there can be mentioned yeast sediment in the bottom of a fermentation tank during the produc tion of an industrial alcohol or a starting alcohol for liquid consumption products, a waste yeast cells formed during the fermenting of wine, and a waste yeast cells formed during the brewing of beer. Among these waste yeast cells, the waste yeast cells obtained during the brewing of beer are most preferred because, (1) the yeast cells are obtained in a large quantity, (2) the yeast cells are obtained in the form of a relatively concentrated suspension for reasons related to the process of brewing beer, and (3) since a starting fermentation liquid used in the brewing of beer is substantially free of solids, waste yeast cells having a small amount of adhering impurities can be obtained in a substantially sterile state.

In the present invention, the yeast cells thus obtained are subjected to an aeration treatment, or an aeration treatment with agitation, to increase the amount of SOD accumulated in the yeast cells. The initial concentration of yeast cells is not particularly critical, and selection of the initial concentration can be made within a broad range. But, since SOD is mainly accumulated in cells, at the step of recovering SOD, advantageously the cells are first collected from the culture medium and SOD is then recovered from the cells. Therefore, preferably the initial concentration of the yeast cells at the aeration treatment or the aeration treatment with agitation is high, so long as the accumulation of SOD is not adversely affected. If the initial concentration is increased, the amount per fermentation volume of SOD accumulated in the cells can be increased while controlling the growth of the yeast. In view of the foregoing, preferably the aeration treatment or the aeration treatment with agitation is initiated at a yeast concentration higher than the yeast concentration (ordinarily lower than $5 \times 10^7$ cells/ml) attained under anaerobic conditions, and especially preferably, the aeration treatment or the aeration treatment with agitation is initiated at a yeast concentration higher than the yeast concentration (ordinarily lower than $1 \times 10^8$ cells/ml) generally attained when the yeast is cultured under aerobic conditions. More specifically, preferably the initial yeast concentration is $10^8$ to $10^9$ cells/ml. In general, in the process of brewing beer, the waste yeast cells are obtained in the form of an aqueous suspension having a concentration of $1 \times 10^9$ to $3 \times 10^9$ cells/ml. Accordingly, the above-mentioned initial concentration of the yeast adopted in the aerobic culture according to the present invention can be easily attained only by diluting the above-mentioned waste yeast suspension with an aqueous liquid.

Preferably, a carbon source, a nitrogen source, an inorganic salt, a trace nutrient, and the like are added to the culture medium (that is, the aqueous yeast suspension) used at the aeration treatment or the aeration treatment with stirring. Any of carbon sources utilized by the yeast used in the present invention can be used. For example, glucose, sucrose and maltose. Any of the nitrogen sources utilized by the yeast used in the present invention can be used. For example, urea, ammonium sulfate and yeast extract are used. As the inorganic salt, appropriate combinations of anions can be used, such as phosphate anion and cations such as magnesium, sodium, manganes, zinc, copper and calcium ions. As the trace nutrient, vitamins such as biotin, vitamin $B_1$ and vitamin $B_2$ and a lipid can be used. The concentrations of the additives are different according to the kinds of the additives. For example, the concentration of the carbon source is preferably 2 to 20%, and the trace nutrient are added at concentrations of 5 to 0.1% for the nitrogen source, 2 to 0.01% for the inorganic salt, and 1 to 0.001% for the trace nutrient.

These additives may be added at once at the start of aeration or aeration with stirring. Alternatively, these additives, especially a carbon source, can be added separately as portions during the aeration or aeration and stirring.

The yeast-containing culture medium for the aeration treatment or the aeration treatment with agitation may be prepared by dissolving predetermined amounts of the above-mentioned nutrient sources into water and diluting the waste yeast suspension with the aqueous solution thus prepared, or by diluting the waste yeast suspension with a predetermined amount of water and dissolving the above-mentioned nutrient sources into the yeast suspension thus prepared. Preferably, the medium is aseptically prepared, but where the aeration treatment or the aeration treatment with agitation is initiated at such a high yeast concentration as described above, the risk of contamination during the treatment is small, and therefore, a strictly sterile operation is not required.

For a small-scale culturing such as flask culturing, aerobic conditions can be attained by shaking the culture medium. In large-scale culturing, aerobic conditions can be attained by aeration or aeration with agitation. This aeration and agitation can be easily accomplished by using an ordinary aerobic fermentation tank. The culturing temperature is within the range customarily adopted for the culture of yeasts, for example, 15 to 40°C, preferably 25 to 35°C. The aeration treatment time is ordinarily 5 to 20 hours. If foaming occurs in the culture medium, for example, at the agitation culturing, a defoamer such as Adecanol (registered trademark; supplied by Asahi Denka Kogyo) can be added.

After the aeration treatment or the aeration treatment with agitation is terminated, SOD is recovered from the cultured medium. In the process of the present invention, SOD is mainly accumulated in cells of the yeast. Accordingly, preferably the cells are first separated from the cultured medium and SOD isolated from the cells and purified. Separation of the cells from the cultured medium can be performed by customary means such as centrifugal separation or filtration. The obtained cells are disrupted for extraction by such means as autolysis, grinding with glass beads, or ultrasonic treatment, and SOD is obtained from the disrupted cells. When the purification of SOD is carried out, separation and purification means known in the art can be appropriately used to obtain a product having a desired purity. Namely, purification can be accomplished by appropriately combining a salting out method using ammonium sulfate or the like, a precipitattion method using an organic solvent, an isoelectric precipitation method, a membrane-using treatment such as the ultrafiltration method, the adsorption method using calcium phosphate or alumina, the ion exchange chromatography method using diethylaminoethyl cellulose or carboxmethyl cellulose, and the gel filtration chromatography method using Sephadex (supplied by Pharmacia) or Ultrogel (supplied by LKB), and a enzyme purified product meeting the intended final use can be obtained.

The measurement of the SOD activity is performed by the cytochrome C method (J.M. McCord & I. Fridovich, J. Biol. Chem., 244, 6049-6055, 1969). More specifically, a reaction mixture (0.4 ml) is prepared in a cell having an optical path of 1 cm so that the final concentrations are 50 mM phosphate buffer (pH 7.8), 0.1 mM sodium ethylenediamine tetraacetate, 10μM ferricytochrome C and 5 μM xanthine, and 50 μl of an SOD solution diluted within a measurable range with phosphate buffer (ph 7.8) is added and 50 μl of a xanthine oxidase solution is added so that the total amount is 0.5 ml. At 25°C, the initial rate (v) of increase of the absorption at 550 nm due to the reduction of ferricytochrome C is measured by a spectrophotometer. Furthermore, phosphate buffer (pH 7.8) is added instead of the enzyme solution and the initial rate (V) of increase of the above absorption is measured. Under these conditions, the amount of SOD required to inhibit the rate of reduction of ferricytochrome C by 50% is defined as 1 unit of activity, and the SOD acitivity can be calculated by the formula (V/v - 1). The protein content is determined by the Lowry method (O.H. Lowry et al, J.Biol. Chem., 193, 265-275, 1951).

According to the method of the present invention, the yeast cells necessary for the production of SOD are prepared under anaerobic conditions, and therefore, the cost of energy necessary for preparing the yeast cells can be reduced. According to a preferred embodiment of the present invention, waste yeast cells obtained during the brewing of beer or the like are used, and therefore, no cost is incurred for the production of the yeast cells, and a by-product formed in the production of beer or the like can be economically utilized.

The present invention will now be described in detail with reference to the following examples.

Example 1

A 50-liter jar fermenter was charged with 15 $\ell$ of a beer waste yeast suspension (1.8 x 10^9 cells/ml) obtained from the Katsura Brewery of Suntory Ltd., and 15 $\ell$ of an aqueous solution containing 10% sucrose, 2% urea, 1% potassium phosphate, 0.4% magnesium sulfate, and 0.1% yeast extract was added. Aeration was carried out at 28°C and 0.9 vvm for 7 hours, and Adecanol was appropriately added for defoaming. After termination of the aeration, cells were collected by centrifugation and and washed with 30 $\ell$ of water, and centrifugation was conducted again to obtain 9.7 kg of a yeast cake. The temperature of the cake was maintained at 25°C, 485 ml of ethyl acetate was added, and the mixture was stirred for 1 hour to lyse the yeast cells. Then, 9.7 $\ell$ of water was added to the obtained lysate and the pH value of the suspension was adjusted to 7.0 by 10N sodium hydroxide, and the suspension was allowed to stand for 20 hours at 25°C to enhance autolysis. The, 14.3 $\ell$ of a supernatant was obtained from the suspension by the centrifugation. This supernatant contained 1.75 x 10^6 units of SOD. The pH value of the solution was reduced to 4.8 by glacial acetic acid, and the denatured and precipitated protein was separated by centrifugation. The obtained supernatant was diluted with 5 volume of wafer and was then concentrated to about 5 $\ell$ by ultrafiltration. The dilution and concentration were conducted 2 times as a whole.

To the above concentrate was added 0.5 $\ell$ of fine granular carboxymethyl cellulose (CM-52 supplied by Wattman) equilibriated at pH 4.8 with 25 mM sodium acetate, and the mixture was stirred for 1 hour, and then transferred into a column having a diameter of 8 cm. Washing was then effected with 2.5 $\ell$ of 25 mM sodium acetate buffer (pH 4.8), and an enzyme-containing fraction was collected with sodium acetate buffer adjusted to pH 4.8 at a linear concentration gradient of from 25 mM to 250 mM. The ion exchange chromatography using CM-52 was carried out according to the method disclosed in Japanese Unexamined Patent Publication No. 56-35983. The obtained total SOD activity was 7.43 x 10^5 units. Ammonium sulfate was added to the obtained solution to a concentration corresponding to 90% of the saturation, and the centrifugation was carried out. Phosphate buffer was then added to the obtained precipitate and the obtained solution passed through a column having a diameter of 1 cm and a length of 100 cm, which was packed with Ultrogel ACA 44 (supplied by LKB) equilibrated with 10 mM phosphate buffer containing 0.1 M sodium chloride. The obtained SOD-active fraction was dialyzed against water and lyophylized to obtain 93 mg of a powder product having a total SOD activity of 7.05 x 10^5 units and a specific activity of 8700 units/mg protein.

Example 2

A 5-liter jar fermenter equipped with aeration and stirring apparatuses was charged with 1.5 $\ell$ of a beer brewing waste yeast suspension (1.8 x 10^9 cells/ml), and 1.5 $\ell$ of an aqueous solution containing 10% sucrose, 2% urea, 1% potassium phosphate, 0.4% magnesium sulfate, and 0.1% yeast extract was added. Incuvation was conducted for 7 hours at 28°C and at various aeration rates. The obtained result is shown in Table 1. Note, the measurement of the SOD activity was made on the supernatant obtained by performing the autolysis of the cultured cells and subjecting the mixture to centrifugation in the same manner as described in Example 1.

## Table 1

| Treatment | Amount (kg) of Wet Yeast Cells | Total SOD Activity ($10^5$ units) | SOD Activity/ Amount of Cells |
|---|---|---|---|
| non-aeration | 1.08 | 0.67 | 0.62 |
| aeration at 0.3 vvm | 0.99 | 1.58 | 1.60 |
| aeration at 0.9 vvm | 0.98 | 1.71 | 1.74 |
| aeration at 1.5 vvm | 1.01 | 1.85 | 1.83 |
| aeration at 2.0 vvm | 0.97 | 0.80 | 0.82 |

As is apparent from the result shown in Table 1, even if a cell suspension having a high cell concentration such that it cannot be adopted for ordinary culturing is used, the content of SOD in the cells can be increased by aeration. In this example, the SOD activity per amount of the cells was high when the aeration rate was 0.3 to 1.5 vvm.

Example 3

The influence of the ratio of dilution of the beer brewing waste yeast suspension was examined.

A beer brewing waste yeast suspension ($1.3 \times 10^9$ cells/ml) was diluted to various yeast concentrations so that the final ingredient concentrations were 1% sucrose, 0.05% potassium phosphate, 0.1% ammonium sulfate, and 0.05% yeast extract. In the same manner as described in Example 2, 3 $\ell$ of the dilution was charged into a 5-liter jar fermenter and aeration was carried out at 28°C and 0.75 vvm. The measurement of the SOD activity was carried out in the same manner as described in Example 2. The obtained result is shown in Table 2.

## Table 2

| Dilution Ratio | Aeration Time (hours) | Amount (kg) of Wet Yeast Cells | Total SOD Activity ($10^5$ units) | Specific Activity of SOD (units/mg protein) |
|---|---|---|---|---|
| 1 | 0 | 1.58 | 1.05 | 4.03 |
| 1 | 5 | 1.41 | 2.18 | 12.3 |
| 2 | 5 | 0.74 | 1.54 | 15.7 |
| 4 | 5 | 0.51 | 1.22 | 17.0 |
| 1 | 20 | 1.55 | 3.46 | 8.6 |
| 2 | 20 | 0.96 | 2.68 | 33.7 |
| 4 | 20 | 0.54 | 1.08 | 64.4 |

From the result shown in Table 2, it is seen that, even if the dilution ratio is 1, that is, even if the starting suspension is not diluted, the SOD content in the cells is increased by aeration, if the starting suspension is diluted at a ratio of 2 to 4, the SOD content in the cells per unit weight of the wet cells is increased and the specific activity of the SOD preparation prominently increased.

**Claims**

1. A process for the preparation of superoxide dismutase by the fermentation method using yeast cells, which comprises subjecting yeast cells grown by culturing under an anaerobic condition to an aeration treatment or an aeration treatment with agitation to increase superoxide dismutase in the cells and recovering the superoxide dismutase.

2. A process according to claim 1, wherein the culturing under the anaerobic condition is carried out as alcohol-forming fermentation.

3. A process according claim 2, wherein the alcohol-forming fermentation is a fermentation for the brewing of beer.

4. A process according to any of claims 1 through 3 wherein the anaerobic condition is attained by non-aeration.

5. A process according to claim 1, wherein the aeration rate is 0.3 to 2.0 vvm.

6. A process according to claim 1, wherein the aeration treatment or the aeration treatment with agitation is initiated at a yeast concentration higher than the yeast concentration attained under the anaerobic condition.

7. A process according to claim 6, wherein the aeration treatment or the aeration treatment with agitation is initiated at a yeast concentration higher than the yeast concentration attained when the culturing of the yeast is carried out under an aerobic condition from the start of the culturing.

8. A process according to claim 7, wherein the yeast concentration at the start of the aeration treatment or the aeration treatment with agitation is $10^8$ to $10^9$ cells/ml.